# EUROPEAN PATENT SPECIFICATION

(11) **EP 2 650 019 B1**
(45) Date of publication and mention of the grant of the patent: **05.08.2015**
(21) Application number: 11846481.7
(22) Date of filing: 06.12.2011
(51) Int. Cl.: A61K 45/00, A61K 9/70, A61K 31/55, A61K 45/08, A61K 47/10, A61K 47/14, A61K 47/32, A61K 47/34

(54) **NORADRENERGIC AND SPECIFIC SEROTONERGIC ANTIDEPRESSANT-CONTAINING TRANSDERMAL PATCH**
NORADRENERGISCHES UND SPEZIFISCH SEROTONERGISCHES ANTIDEPRESSIVUMHALTIGES HAUTPFLASTER
TIMBRE TRANSDERMIQUE CONTENANT UN ANTIDÉPRESSEUR NORADRÉNERGIQUE ET SÉROTONINERGIQUE SPÉCIFIQUE

(30) Priority: 07.12.2010 JP 2010272098; 07.12.2010 JP 2010272099
(43) Date of publication of application: 16.10.2013
(73) Proprietor: Yutoku Pharmaceutical Industries Co., Ltd., Kashima-shi, Saga 849-1393 (JP)
(72) Inventor: YOSHITAKE, Makoto, Kashima-shi Saga 849-1393 (JP); OGAWA, Takayuki, Kashima-shi Saga 849-1393 (JP); YOSHITAKE, Takashi, Stockholm 11523 (SE)
(74) Representative: Blodig, Wolfgang
(86) International application number: PCT/JP2011/078114
(87) International publication number: WO 2012/077651

(56) References cited:
- EP-A2- 2 233 158
- WO-A1-2009/041122
- WO-A1-2009/055860
- WO-A1-2010/126124
- JP-A- 2005 510 488
- JP-A- 2009 531 452
- JP-A- 2010 163 367

## Description

### Technical Field

The present invention relates to a transdermal patch comprising a support, a drug-containing layer and a release liner, in which the drug-containing layer contains, as the active ingredient, a noradrenergic and specific serotonergic antidepressant (NaSSA).

### Background Art

NaSSA exhibits an antagonistic effect to central presynaptic α2 adrenalin autoreceptor and heteroreceptor, and is a type of antidepressant having an effect of enhancing the neurotransmission of both central serotonin (5-HT) and noradrenalin (NA). This NaSSA has a functional mechanism different from that of other antidepressants such as a selective serotonin reuptake inhibitor (SSRI), for example, fluvoxamine and a serotonin/norepinephrine reuptake inhibitor (SNRI), for example, milnacipran, and is considered to promote release of 5-HT and NA to thereby exhibit the antidepressant effect on a level equivalent to or higher than that of SSRI and SNRI. Mirtazapine that is the sole commercially available pharmaceutical ingredient as NaSSA is characterized by its ability to not only promote release of 5-HT and NA but also to promote release of dopamine (DA). At present, mirtazapine is used as a therapeutic medicine for depression and anxiety disorder but only in the form of an oral preparation. Further, mirtazapine is under clinical research as a therapeutic medicine for fibromyalgia, etc.

As compared with SSRI and others, NaSSA has few side effects such as nausea and vomiting, sexual dysfunction, but may express sedative side effects such as somnolentia, dry mouth and fatigue due to the strong antihistamine effect and muscarinic anticholinergic effect, and is therefore known to be problematic in terms of stabilizing the blood level and sustaining its effect.

On the other hand, recently, external preparations containing a centrally-acting pharmaceutical agent such as an antidepressant have been developed. For example, there are disclosed transdermal patches containing SSRI such as fluoxetine, sertraline, fluvoxamine or paroxetine (Patent Document 1, Patent Document 2). Whilst these documents evidence transdermal absorption of SSRI, inhibition of skin irritation by SSRI, and inhibition of side effects owing to rapid blood level increase achieved by transdermal patches containing SSRI. They do not disclose a NaSSA-containing transdermal patch or its composition.

As described above, NaSSA and SSRI differ in the functional mechanism and the side effects thereof. There is no disclosure of the usefulness and the safety of a NaSSA-containing transdermal patch, not to mention the relationship between the blood level and the potency in administration of a NaSSA-containing transdermal patch.

Moreover, in the related art, there is no suggestion of an external preparation of a type which enables transdermal absorption of a sufficient amount of NaSSA for expressing the therapeutical effect thereof and its usefulness.

JP 2005 510488 A relates to breathable patches for topically delivering local anaesthetics to treat or prevent pain.

WO 2009/055860 A1 relates to a transdermal delivery system and to a method of transdermal delivery of a physiologically active agent.

WO 2010/126124 A1 relates to a transdermal preparation which is capable of long-term release of a basic drug from a preparation. The transdermal preparation comprises a substrate and an adhesive layer containing a basic drug and a water soluble polymer.

JP 2010 163367 A relates to patches and transdermal plaster preparation with easy operation and a good handling property at the time of pasting to the skin.

WO 2009/041122 A1 relates to a patch material comprising a layer structure having a base material layer provided on its one major surface with an adhesive layer.

JP 2009 531452 A relates to a process for the preparation of a hot-melt extruded laminate.

EP 2 233 158 A2 relates to a patch and a patch preparation showing appropriate skin adhesiveness and flexibility.
Patent Document 1: JP-A 2006-335714
Patent Document 2: JP-A 2007-284378

### Disclosure of Invention

### Technical Problem

An object of the present invention is to provide a NaSSA-containing transdermal patch, and more specifically to provide a NaSSA-containing transdermal patch capable of inhibiting the expression of side effects that are problematic in oral administration, enabling continuous administration of a therapeutically or preventively effective amount of NaSSA into living bodies and in which the effectiveness of NaSSA is improved.

### Means for solving the problem

For solving the above-mentioned problems, the present inventors have repeatedly and assiduously made researches and, as a result, have developed a novel, NaSSA-containing transdermal patch. The inventors have found that the above-mentioned problems can be solved by a transdermal patch which comprises a support, a drug-containing layer and a release liner as defined in claim 1.

Further, the inventors have confirmed for the first time that, in the transdermal patch containing mirtazapine or a salt thereof as NaSSA, the active ingredient undergoes oxidative decomposition after preparation of the patch, especially in the presence of oxygen to form related substances therein. Therefore, the inventors have repeatedly and assiduously made researches in order to provide a NaSSA-containing transdermal patch that has improved NaSSA stability and, as a result, have found that, when a specific antioxidant is incorporated in the composition, then the formation of the related substances can be prevented and the temporal drug stability can be thereby enhanced.

### Effects of Invention

The present invention is a novel, NaSSA-containing transdermal patch. The patch is a novel transdermal patch which, when applied to the skin of a patient, has enabled for the first time continuous administration of a therapeutically or preventively effective amount of NaSSA into living bodies while preventing the expression of side effects that are problematic in oral preparations. Another advantage of the transdermal patch of the invention is that the dose and the blood level of NaSSA can be controlled and therefore the active ingredient can be readily administered in an amount necessary for expression of the pharmacological potency thereof but smaller than the level thereof to express side effects. Even when any undesirable effects are expressed, administration can be immediately stopped by just removing the patch. Accordingly, the transdermal patch of the invention is an excellent preparation in terms of safety as compared with oral preparations, etc. Further, like oral antidepressants, the transdermal patch of the invention can be used favorably as differentiated from any other transdermal patches containing an antidepressant that has a different functional mechanism such as SSRI, depending on the degree of the depression of a patient, the compatibility between the patient and the pharmaceutical agent, and the expression state of side effects; and consequently, the transdermal patch of the invention broadens the latitude in selecting pharmaceutical preparations in treatment for depression, and is therefore therapeutically useful.

### Brief Description of Drawings

[Fig. 1]
   This is a graph showing a brain monoamine (NA, DA and 5-HT) level change in administration of a predetermined dose of a 3 mg/kg mirtazapine solution for 12 hours.
[Fig. 2]
   This is a graph showing a rat plasma mirtazapine level change from the patches obtained in Example 8, Example 12, Example 18, Example 19, Example 20 and Example 21.

### Detailed Description of Embodiments

The transdermal patch of the invention comprises a drug-containing layer formed on the support thereof. Until use, a release liner is kept provided on the drug-containing layer for the purpose of protecting the drug-containing layer.

The drug-containing layer contains the NaSSA mirtazapine as the active ingredient therein, of which the past record of actual use for treatment for depression and anxiety disorder as oral preparations has been verified and of which clinical studies as therapeutic medicines for pain of fibromyalgia are being promoted, and which is relatively easily available.

The transdermal patch of the invention contains a therapeutically effective amount of NaSSA. For long-term continuous transdermal administration of the active ingredient to a patient suffering from depression, anxiety disorder or fibromyalgia, in an amount effective for treatment for depression, anxiety disorder or fibromyalgia and in an amount expressing few side effects, it is important to incorporate a predetermined amount of the active ingredient in the drug-containing layer.

In the description of the present invention, the transdermal patch means an adhesive patch for medical treatment, which, when stuck to skin, releases a therapeutically effective amount of the active ingredient to pass through the skin to reach the blood.

The content of the active ingredient in the transdermal patch of the invention is within a range of from 2 to 30% by mass, preferably from 3 to 25% by mass relative to the drug-containing layer. The content of less than 2% by mass is unfavorable, since a sufficient increase in the brain monoamine level could not be recognized. On the other hand, the content of more than 30% by mass is also unfavorable, since it is difficult to keep good physical properties of the patch.

The active ingredient to be contained in the transdermal patch of the invention may be in the form of a pharmaceutically-acceptable salt thereof. In the case of mirtazapine, acid addition salts with inorganic acids or organic acids may be mentioned as, but not limited to, the pharmaceutically-acceptable salts of mirtazapine, including hydrochloride, hydrobromide, nitrate, phosphate, sulfate, acetate, ascorbate, benzoate, cinnamate, citrate, formate, fumarate, glutamate, lactate, maleate, malate, malonate, mandelate, methanesulfonate (mesilate), phthalate, salicylate, stearate, succinate, tartrate, propionate, butyrate, pamonate, p-toluenesulfonate (tosylate), etc. In the invention, a free form of mirtazapine is preferably used.

The transdermal patch of the invention comprises a support, a drug-containing layer and a release liner. Further, if desired, a release control film may be added to the skin-application side of the drug-containing layer for controlling the transdermal absorption of the active ingredient through the skin, and an adhesive layer may be added thereto for applying the patch to skin. Moreover, a reservoir-type patch may also be employed.

In the transdermal patch of the invention, the drug-containing layer is a matrix-type adhesive layer that contains the active ingredient and, as the base ingredient thereof, an adhesive ingredient. The patch of the invention is a matrix-type patch that contains such a matrix-type adhesive layer, the preparation for the patch can be easily manufactured and the patch does not require any additional layer such as an adhesive layer, and consequently, the production cost for the patch can be reduced.

The adhesive ingredient to be contained in the drug-containing layer of the transdermal patch of the invention is preferably a nonaqueous adhesive ingredient, including a rubber-type adhesive ingredient, an acrylic polymer and a silicone polymer.

The rubber-type adhesive ingredient includes one or more selected from styrene-isoprene-styrene block copolymer, styrene-butadiene-styrene block copolymer, styrene-butadiene rubber, polyisobutylene, polybutene, butyl rubber, natural rubber and isoprene rubber; and any of these may be used here.

The acrylic polymer includes, though not defined thereto, polymers or copolymers containing, as the monomer unit thereof, at least one (meth)acrylate of typically 2-ethylhexyl acrylate, methyl acrylate, butyl acrylate, 2-hydroxyethyl acrylate or 2-ethylhexyl methacrylate. For example, herein usable are adhesives of acrylic polymers that contain acrylic acid/octyl acrylate copolymer, 2-ethylhexyl acrylate/N-vinyl-2-pyrrolidone/1,6-hexaneglycol dimethacrylate copolymer, 2-ethylhexyl acrylate/vinyl acetate copolymer, 2-ethylhexyl acrylate/vinyl acetate/acrylic acid copolymer, 2-ethylhexyl acrylate/2-ethylhexyl methacrylate/dodecyl methacrylate copolymer, methyl acrylate/2-ethylhexyl acrylate copolymer resin emulsion, or acrylic resin alkanolamine liquid; and for example, usable are commercially-available DURO-TAK™ acrylic adhesive series (available from Henkel Technologies Japan), GELVA™ acrylic adhesive series (by Monsanto), SK-DYNE MATRIDERM (by Soken Chemical), EUDRAGIT™ series (by Higuchi Shokai), etc.

The silicone polymer includes polysiloxane derivatives (for example, silicone polymers such as polydimethylsiloxane, amine-resistant polydimethylsiloxane, etc.), etc.

The amount of the adhesive ingredient to be incorporated in the drug-containing layer is from 65 to 98% by mass, preferably from 70 to 97% by mass relative to the drug-containing layer, in consideration of formation of the drug-containing layer and of sufficient drug releasability.

As the adhesive ingredient to be contained in the drug-containing layer of the transdermal patch of the invention, one or more may be selected from the above-mentioned rubber-type adhesive ingredients, acrylic polymers and silicone polymers.

For improving the transdermal absorption of the active ingredient, if desired, a transdermal absorption promoter may be incorporated. The transdermal absorption promoter may be any compound that has heretofore been recognized to exhibit an absorption-promoting effect in transdermal administration, and includes, for example, alkanolamines such as diisopropanolamine, triisopropanolamine, etc., fatty acids or their esters such as lauric acid, oleic acid, isopropyl myristate, octyldodecyl myristate, oleic acid glycerol monoester, hexadecyl isostearate, etc.; alcohols or their esters or ethers such as oleyl alcohol, propylene glycol, propylene glycol monocaprylate, polyethylene glycol monooleate, etc. ; sorbitan esters or ethers such as sorbitan monolaurate, sorbitan monooleate, etc.; polyoxyethylene sorbitan fatty acid esters such as polyoxyethylene sorbitan monooleate, polyoxyethylene sorbitan monopalmitate, etc.; phenol ethers such as polyoxyethylene nonylphenyl ether, polyoxyethylene octylphenyl ether, etc.; castor oil or hardened castor oil; ionic surfactants such as oleoyl sarcosine, lauryldimethylaminoacetate betaine, sodium laurylsulfate, etc.; nonionic surfactants such as polyoxyethylene oleyl ether, polyoxyethylene lauryl ether, dimethyllaurylamine oxide, etc.; alkylmethyl sulfoxides such as dimethyl sulfoxide, decylmethyl sulfoxide, etc.; pyrrolidones such as 2-pyrrolidone, 1-methyl-2-pyrrolidone, etc.; azacycloalkanes such as 1-dodecylazacycloheptan-2-one, 1-geranylazacycloheptan-2-one, etc.; terpenes such as menthol, camphor, limonene, etc. Of those, preferred are myristates such as isopropyl myristate, sebacates such as diisopropyl sebacate, etc.; menthol, polyoxyethylene oleyl ether or Polysorbate 80™.

The amount of the transdermal absorption promoter, if any, in the transdermal patch of the invention may be within a range of from 0.1 to 15% by mass, preferably from 1 to 10% by mass relative to the drug-containing layer. The amount of more than 15% by mass is unfavorable since there may occur skin irritation derived from the transdermal absorption promoter and the physical properties of the preparation may deteriorate and cause stickiness.

An antioxidant is incorporated in the transdermal patch of the invention. The antioxidant includes phenolic antioxidants, ascorbic acid and its ester derivatives, sodium sulfite, sodium hydrogensulfite, sodium pyrosulfite, sodium edetate, citric acid, potassium dichloroisocyanurate, soybean lecithin, thymol, tocopherol and its ester derivatives, 1,3-butylene glycol, benzotriazole, monothioglycerol, 2-mercaptobenzimidazole and its salts, etc. Of those, preferred are ascorbic acid and its esters, or phenolic antioxidants.

As ascorbic acid and its salts, there are mentioned L-ascorbic acid, L-ascorbyl palmitate, L-ascorbyl stearate, L-ascorbic acid 2-glucoside, sodium L-ascorbate, calcium L-ascorbate, magnesium L-ascorbyl phosphate, isoascorbic acid, sodium isoascorbate, etc. Above all, preferred are L-ascorbyl palmitate and isoascorbic acid.

The phenolic antioxidant includes dibutylhydroxytoluene, butylhydroxyanisole, propyl gallate, octyl gallate, lauryl gallate, nordihydroguairaretic acid, trihydroxybutyrophenone, tert-butylhydroquinone, 4-hydroxymethyl-2,6-di-tert-butylphenol, etc. Above all, preferred is dibutylhydroxytoluene.

In some Examples of the invention, there was confirmed the presence of NaSSA-related substances in the drug-containing layer of the transdermal patch, or the formation of those related substances with time even in a case where the substances did not exist immediately after the production of the transdermal patch. Here, the NaSSA-related substances are substances derived from NaSSA (substances not recognized in a NaSSA-free adhesive layer). Specific examples of the NaSSA-related substances include those detected in a retention time of about 7 minutes in analysis of the patch of the invention under the analysis condition described in Examples given below (hereinafter referred to as "related substances 1") and those detected in a retention time of about 11 minutes therein (hereinafter referred to as "related substances 2"). In the invention, by incorporating an antioxidant in the patch, the formation of the related substances 1 and the related substances 2 can be prevented and the total production of the related substances can be thereby reduced.

If desired, the transdermal patch of the invention may contain any other additional ingredients such as a plasticizer, a crosslinking agent, a colorant, a UV absorbent, a tackifier, etc.

The plasticizer includes petroleum oils such as paraffinic process oil, naphthenic process oil, aromatic process oil, etc.; liquid fatty acid esters such as isopropyl myristate, hexyl laurate, diethyl sebacate, diisopropyl sebacate, isopropyl linoleate, etc.; vegetable oils such as olive oil, camellia oil, castor oil, tall oil, peanut oil, etc.; glycerin, chlorobutanol, vinyl acetate resin, dimethylpolysiloxane-silicon dioxide mixture, D-sorbitol, middle-chain fatty acid triglyceride, triacetin, 2-pyrrolidone, phytosterol, propylene glycol, polyethylene glycol, Polysorbate 80™, glycerin monostearate, etc.

The crosslinking agent includes thermosetting resins such as amino resin, phenolic resin, epoxy resin, alkyd resin, unsaturated polyester, etc.; isocyanate compounds, organic crosslinking agents; inorganic crosslinking agents such as metals or metal compounds, etc.

The colorant includes indigocarmine, yellow iron oxide, yellow iron sesquioxide, carbon black, caramel, Photopigment 201, dwarf bamboo extract, black iron oxide, dragon's blood palm, zinc oxide, titanium oxide, iron sesquioxide, amaranth, sodium hydroxide, talc, sodium copper chlorophyllin, hull-less barley green leaf extract powder, d-borneol, octyldodecyl myristate, methylrosanilinium chloride, methylene blue, ammonium manganese phosphate, rose oil, etc.

The UV absorbent includes amino acid compounds such as urocanic acid, etc.; benzophenone compounds such as 2,4-dihydroxybenzophenone, 2-hydroxy-4-n-octoxybenzophenone, etc.; citric acid derivatives such as cinoxate, p-methoxycinnamic acid diethanolamine, etc.; cyanoacrylate derivatives such as 2-ethylhexyl-2-cyano-3,3'-diphenyl acrylate, etc.; p-aminobenzoic acid derivatives such as ethyl p-aminobenzoate, propyl p-aminobenzoate, etc.; anthranilic acid derivatives such as menthyl anthranilate, etc.; salicylic acid derivatives such as phenyl salicylate, p-octylphenyl salicylate, etc.; coumarin derivatives such as 7-ethylamino-4-methylcoumarin, 7,8-dihydroxycoumarin, etc.

The tackifier includes rosin derivatives such as rosin, rosin glycerin ester, hydrogenated rosin, hydrogenated rosin glycerin ester, etc.; alicyclic saturated hydrocarbon resins, alicyclic hydrocarbon resins, terpene resins, aliphatic saturated hydrocarbon resins, aliphatic hydrocarbon resins, resin maleate, carnauba wax, sodium carmellose, xanthane gum, chitosan, glycerin, magnesium aluminium silicate, light anhydrous silicic acid, benzyl acetate, talc, hydroxyethyl cellulose, hydroxypropyl cellulose, hypromellose, polyacrylic acid, sodium polyacrylate, partially-neutralized polyacrylic acid, polyvinyl alcohol, etc.

As the support of the transdermal patch of the invention, a drug-impervious elastic or nonelastic support may be used. The support of the type includes, for example, synthetic resin films or sheets or their laminates of polyethylene, polypropylene, polybutadiene, ethylene-vinyl acetate copolymer, polyvinyl chloride, polyester (polyethylene terephthalate, etc.), nylon, polyurethane, etc. ; porous substances, foams, papers, woven fabrics, nonwoven fabrics, etc.

As the release liner of the transdermal patch of the invention, a drug-impervious release liner may be used. The release liner includes, for example, films formed of a polymer material such as polyethylene, polypropylene or polyester; those produced through vapor deposition of aluminium on a film; those produced by coating paper with silicone oil, etc. Above all, preferred are polyester films since the active ingredient does not permeate through them and from the viewpoint of the workability and the low cost thereof; and more preferred are polyethylene terephthalate (PET) films. Further, as the release liner, also usable is a laminate film produced through lamination of multiple materials, etc.

There is no specific limitation on the transdermal patch of the invention and it can be produced according to any known production method. Preferred known production methods for the transdermal patch of the invention include a method that comprises, for example, dissolving an active ingredient and an adhesive and optionally a transdermal absorption promoter in an organic solvent of ethyl acetate, hexane, toluene or a mixed solvent thereof, then spreading the dissolved matter onto a release liner or a support, evaporating away the solvent from the dissolved matter to form a drug-containing layer, and thereafter sticking a support or a release liner thereto to give a transdermal patch; a method that comprises melting an active ingredient and an adhesive and optionally a transdermal absorption promoter under heat, then spreading the resulting melt onto a release liner or a support to form a drug-containing layer thereon, and thereafter sticking a support or a release liner thereto to give a transdermal patch, etc.

### Examples

The present invention is described in more detail with reference to the following Examples, not being limited thereto.

### Example 1

According to the blend ratio shown in Table 1, styrene-isoprene-styrene block copolymer, polybutene, hydrogenated rosin glycerin ester and dibutylhydroxytoluene were stirred and mixed in toluene, and then mirtazapine was added thereto, and stirred and mixed to give a uniform dissolved matter. Next, using a doctor knife coater, the dissolved matter was spread on a release film (silicone-processed PET film, Fujimori Industry) to have a thickness of 100 µm after solvent removal, then the solvent was evaporated away to form a drug-containing layer, and thereafter a support was stuck thereto.

### Example 2

A transdermal patch was produced in the same manner as in Example 1, except that the blend ratio of styrene-isoprene-styrene block copolymer, polybutene, hydrogenated rosin glycerin ester and dibutylhydroxytoluene was changed as in Table 1.

### Example 3

According to the blend ratio shown in Table 1, styrene-isoprene-styrene block copolymer, polybutene and hydrogenated rosin glycerin ester were stirred and mixed in toluene, then L-ascorbyl palmitate dissolved in ethanol was added thereto and further stirred and mixed, and thereafter mirtazapine was added thereto, and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto.

### Example 4

According to the blend ratio shown in Table 1, styrene-isoprene-styrene block copolymer, polybutene, hydrogenated rosin glycerin ester and dibutylhydroxytoluene were stirred and mixed in toluene, then L-ascorbyl palmitate dissolved in ethanol was added thereto and further stirred and mixed, and thereafter mirtazapine was added thereto, and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto.

### Example 5

A transdermal patch was produced in the same manner as in Example 3 and according to the blend ratio as in Table 1, except that isoascorbic acid was used in place of L-ascorbyl palmitate.

### Example 6

According to the blend ratio shown in Table 1, styrene-isoprene-styrene block copolymer, polybutene, hydrogenated rosin glycerin ester and tocopherol were stirred and mixed in toluene, then mirtazapine was added thereto, and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto.

### Example 7

According to the blend ratio shown in Table 1, styrene-isoprene-styrene block copolymer, polybutene, hydrogenated rosin glycerin ester, alicyclic saturated hydrocarbon resin and dibutylhydroxytoluene were stirred and mixed in toluene, then L-ascorbyl palmitate dissolved in ethanol was added thereto and further stirred and mixed, and thereafter mirtazapine was added thereto, and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto.

### Example 8

According to the blend ratio shown in Table 1, styrene-isoprene-styrene block copolymer, polybutene, hydrogenated rosin glycerin ester, alicyclic saturated hydrocarbon resin and dibutylhydroxytoluene were stirred and mixed in toluene, then L-ascorbyl palmitate dissolved in ethanol was added thereto and further stirred and mixed, and thereafter mirtazapine and Polysorbate 80™ were added thereto, and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto.

### Example 9

According to the blend ratio shown in Table 1, dibutylhydroxytoluene was added to and stirred with acrylic polymer (DURO-TAK™ 87-4287, obtained from Henkel Technologies Japan), and thereafter mirtazapine was added thereto, and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto. Subsequently, this was cut in a desired size to give a transdermal patch.

### Example 10

A transdermal patch was produced in the same manner as in Example 9 and according to the blend ratio as in Table 1, except that DURO-TAK™ 87-9301 (obtained from Henkel Technologies Japan) was used in place of DURO-TAK™ 87-4287.

### Example 11

A transdermal patch was produced in the same manner as in Example 9 and according to the blend ratio as in Table 2, except that DURO-TAK™ 87-2525 (obtained from Henkel Technologies Japan) was used in place of DURO-TAK™ 87-4287.

### Example 12

According to the blend ratio shown in Table 2, dibutylhydroxytoluene was added to and stirred with DURO-TAK™ 87-4287, and thereafter mirtazapine and isopropyl myristate were added thereto, and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto. Subsequently, this was cut in a desired size to give a transdermal patch.

### Example 13

A transdermal patch was produced in the same manner as in Example 12 and according to the blend ratio as in Table 2, except that diisopropyl sebacate was used in place of isopropyl myristate.

### Example 14

A transdermal patch was produced in the same manner as in Example 12, except that menthol was used in place of isopropyl myristate and that the amount of DURO-TAK™ 87-4287 was changed according to the blend ratio as in Table 2.

### Example 15

A transdermal patch was produced in the same manner as in Example 14 and according to the blend ratio as in Table 2, except that polyoxyethylene oleyl ether was used in place of menthol.

### Example 16

A transdermal patch was produced in the same manner as in Example 12, except that the amount of DURO-TAK™ 87-4287 and that of isopropyl myristate were changed according to the blend ratio as in Table 2.

### Example 17

A transdermal patch was produced in the same manner as in Example 13, except that the amount of DURO-TAK™ 87-4287 and that of diisopropyl sebacate were changed according to the blend ratio as in Table 2.

### Example 18

According to the blend ratio shown in Table 2, styrene-isoprene-styrene block copolymer, polybutene, alicyclic saturated hydrocarbon resin and dibutylhydroxytoluene were stirred and mixed in toluene, then L-ascorbyl palmitate dissolved in ethanol was added thereto and further stirred and mixed, and thereafter mirtazapine and Polysorbate 80™ were added thereto, and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto.

### Example 19

A transdermal patch was produced in the same manner as in Example 18 except that the blend ratio of mirtazapine, styrene-isoprene-styrene block copolymer, polybutene and alicyclic saturated hydrocarbon resin was changed as in Table 2.

### Example 20

According to the blend ratio shown in Table 2, dibutylhydroxytoluene was added to and stirred with acrylic polymer (DURO-TAK™ 87-202A, obtained from Henkel Technologies Japan) and thereafter mirtazapine was added thereto, and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto. Subsequently, this was cut in a desired size to give a transdermal patch.

### Example 21

A transdermal patch was produced in the same manner as in Example 20, except that the amount of mirtazapine and that of DURO-TAK™ 87-202A were changed according to the blend ratio as in Table 3.

### Example 22

According to the blend ratio shown in Table 3, L-ascorbyl palmitate dissolved in ethanol was added to and stirred with DURO-TAK™ 87-202A, then mirtazapine and polyoxyethylene (6) sorbitan monooleate were added thereto and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto. Subsequently, this was cut in a desired size to give a transdermal patch.

### Example 23

A transdermal patch was produced in the same manner as in Example 22 and according to the blend ratio as in Table 3, except that propylene glycol monocaprylate was used in place of polyoxyethylene (6) sorbitan monooleate.

### Example 24

According to the blend ratio shown in Table 3, L-ascorbyl palmitate dissolved in ethanol was added to and stirred with DURO-TAK™ 87-202A, then mirtazapine, isopropyl myristate, polyoxyethylene oleyl ether and caprylic acid were added thereto and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto. Subsequently, this was cut in a desired size to give a transdermal patch.

### Example 25

According to the blend ratio shown in Table 3, L-ascorbyl palmitate and sodium sulfite dissolved in ethanol were added to and stirred with DURO-TAK™ 87-202A, then mirtazapine, isopropyl myristate, polyoxyethylene oleyl ether and caprylic acid were added thereto and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto. Subsequently, this was cut in a desired size to give a transdermal patch.

### Example 26

A transdermal patch was produced in the same manner as in Example 25 except that the amount of DURO-TAK™ 87-202A and that of sodium sulfite were changed according to the blend ratio as in Table 3.

### Example 27

According to the blend ratio shown in Table 3, 2-mercaptobenzimidazole was added to and stirred with DURO-TAK™ 87-202A, then mirtazapine, isopropyl myristate, polyoxyethylene oleyl ether and caprylic acid were added thereto and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto. Subsequently, this was cut in a desired size to give a transdermal patch.

### Example 28

A transdermal patch was produced in the same manner as in Example 27 except that the amount of DURO-TAK™ 87-202A and that of 2-mercaptobenzimidazole were changed according to the blend ratio as in Table 3.

### Comparative Example 1

A transdermal patch was produced in the same manner as in Example 1 except that dibutylhydroxytoluene was omitted and that the blend ratio of styrene-isoprene-styrene block copolymer, polybutene and hydrogenated rosin glycerin ester was changed as in Table 3.

**[Table 1]**

| | Example 1 | Example 2 | Example 3 | Example 4 | Example 5 | Example 6 | Example 7 | Example 8 | Example 9 | Example 10 |
|---|---|---|---|---|---|---|---|---|---|---|
| Mirtazapine | 10.00 | 10.00 | 10.00 | 10.00 | 10.00 | 10.00 | 10.00 | 10.00 | 10.00 | 10.00 |
| Styrene-isoprene-styrene block copolymer | 35.96 | 35.60 | 35.92 | 35.52 | 35.92 | 36.00 | 35.59 | 33.59 | 0.00 | 0.00 |
| Polybutene | 8.99 | 8.90 | 8.98 | 8.88 | 8.98 | 9.00 | 8.90 | 8.40 | 0.00 | 0.00 |
| Hydrogenated rosin glycerin ester | 44.95 | 44.50 | 44.90 | 44.40 | 44.90 | 44.99 | 29.63 | 27.97 | 0.00 | 0.00 |
| Alicyclic saturated hydrocarbon resin | 0.00 | 0.00 | 0.00 | 0.00 | 0.00 | 0.00 | 14.86 | 14.02 | 0.00 | 0.00 |
| DURO-TAK 87-4287 | 0.00 | 0.00 | 0.00 | 0.00 | 0.00 | 0.00 | 0.00 | 0.00 | 89.00 | 0.00 |
| DURO-TAK 87-9301 | 0.00 | 0.00 | 0.00 | 0.00 | 0.00 | 0.00 | 0.00 | 0.00 | 0.00 | 89.00 |
| Polysorbate 80 | 0.00 | 0.00 | 0.00 | 0.00 | 0.00 | 0.00 | 0.00 | 5.00 | 0.00 | 0.00 |
| Dibutylhydroxytoluene | 0.10 | 1.00 | 0.00 | 1.00 | 0.00 | 0.00 | 1.00 | 1.00 | 1.00 | 1.00 |
| Ascorbyl palmitate | 0.00 | 0.00 | 0.20 | 0.20 | 0.00 | 0.00 | 0.02 | 0.02 | 0.00 | 0.00 |
| Isoascorbic acid | 0.00 | 0.00 | 0.00 | 0.00 | 0.20 | 0.00 | 0.00 | 0.00 | 0.00 | 0.00 |
| Tocopherol | 0.00 | 0.00 | 0.00 | 0.00 | 0.00 | 0.01 | 0.00 | 0.00 | 0.00 | 0.00 |

**[Table 2]**

| | Example 11 | Example 12 | Example 13 | Example 14 | Example 15 | Example 16 | Example 17 | Example 18 | Example 19 | Example 20 |
|---|---|---|---|---|---|---|---|---|---|---|
| Mirtazapine | 10.00 | 10.00 | 10.00 | 10.00 | 10.00 | 10.00 | 10.00 | 2.00 | 3.00 | 25.00 |
| Styrene-isoprene-styrene block copolymer | 0.00 | 0.00 | 0.00 | 0.00 | 0.00 | 0.00 | 0.00 | 36.79 | 36.39 | 0.00 |
| Polybutene | 0.00 | 0.00 | 0.00 | 0.00 | 0.00 | 0.00 | 0.00 | 9.20 | 9.10 | 0.00 |
| Alicyclic saturated hydrocarbon resin | 0.00 | 0.00 | 0.00 | 0.00 | 0.00 | 0.00 | 0.00 | 45.99 | 45.49 | 0.00 |
| DURO-TAK 87-4287 | 0.00 | 79.00 | 79.00 | 84.00 | 84.00 | 88.00 | 88.00 | 0.00 | 0.00 | 0.00 |
| DURO-TAK 87-2525 | 89.00 | 0.00 | 0.00 | 0.00 | 0.00 | 0.00 | 0.00 | 0.00 | 0.00 | 0.00 |
| DURO-TAK 87-202A | 0.00 | 0.00 | 0.00 | 0.00 | 0.00 | 0.00 | 0.00 | 0.00 | 0.00 | 74.00 |
| Polysorbate 80 | 0.00 | 0.00 | 0.00 | 0.00 | 0.00 | 0.00 | 0.00 | 5.00 | 5.00 | 0.00 |
| Isopropyl myristate | 0.00 | 10.00 | 0.00 | 0.00 | 0.00 | 1.00 | 0.00 | 0.00 | 0.00 | 0.00 |
| Diisopropyl sebacate | 0.00 | 0.00 | 10.00 | 0.00 | 0.00 | 0.00 | 1.00 | 0.00 | 0.00 | 0.00 |
| Menthol | 0.00 | 0.00 | 0.00 | 5.00 | 0.00 | 0.00 | 0.00 | 0.00 | 0.00 | 0.00 |
| Polyoxyethylene oleyl ether | 0.00 | 0.00 | 0.00 | 0.00 | 5.00 | 0.00 | 0.00 | 0.00 | 0.00 | 0.00 |
| Dibutylhydroxytoluene | 1.00 | 1.00 | 1.00 | 1.00 | 1.00 | 1.00 | 1.00 | 1.00 | 1.00 | 1.00 |
| Ascorbyl palmitate | 0.00 | 0.00 | 0.00 | 0.00 | 0.00 | 0.00 | 0.00 | 0.02 | 0.02 | 0.00 |

**[Table 3]**

| | Example 21 | Example 22 | Example 23 | Example 24 | Example 25 | Example 26 | Example 27 | Example 28 | Comparative Example 1 |
|---|---|---|---|---|---|---|---|---|---|
| Mirtazapine | 30.00 | 10.00 | 10.00 | 10.00 | 10.00 | 10.00 | 10.00 | 10.00 | 10.00 |
| Styrene-isoprene-styrene block copolymer | 0.00 | 0.00 | 0.00 | 0.00 | 0.00 | 0.00 | 0.00 | 0.00 | 36.00 |
| Polybutene | 0.00 | 0.00 | 0.00 | 0.00 | 0.00 | 0.00 | 0.00 | 0.00 | 9.00 |
| Hydrogenated rosin glycerin ester | 0.00 | 0.00 | 0.00 | 0.00 | 0.00 | 0.00 | 0.00 | 0.00 | 45.00 |
| DURO-TAK 87-202A | 69.00 | 82.50 | 82.50 | 79.50 | 79.49 | 79.45 | 79.90 | 79.00 | 0.00 |
| Isopropyl myristate | 0.00 | 0.00 | 0.00 | 3.00 | 3.00 | 3.00 | 3.00 | 3.00 | 0.00 |
| Polyoxyethylene oleyl ether | 0.00 | 0.00 | 0.00 | 2.00 | 2.00 | 2.00 | 2.00 | 2.00 | 0.00 |
| Polyoxyethylene (6) sorbitan monooleate | 0.00 | 7.00 | 0.00 | 0.00 | 0.00 | 0.00 | 0.00 | 0.00 | 0.00 |
| Propylene glycol monocaprylate | 0.00 | 0.00 | 7.00 | 0.00 | 0.00 | 0.00 | 0.00 | 0.00 | 0.00 |
| Dibutylhydroxytoluene | 1.00 | 0.00 | 0.00 | 0.00 | 0.00 | 0.00 | 0.00 | 0.00 | 0.00 |
| Ascorbyl palmitate | 0.00 | 0.50 | 0.50 | 0.50 | 0.50 | 0.50 | 0.00 | 0.00 | 0.00 |
| Sodium sulfite | 0.00 | 0.00 | 0.00 | 0.00 | 0.01 | 0.05 | 0.00 | 0.00 | 0.00 |
| Mercaptobenzimidazole | 0.00 | 0.00 | 0.00 | 0.00 | 0.00 | 0.00 | 0.10 | 1.00 | 0.00 |
| Caprylic acid | 0.00 | 0.00 | 0.00 | 5.00 | 5.00 | 5.00 | 5.00 | 5.00 | 0.00 |

### Test Example 1

### Drug Transdermal Permeation Test:

Using a transmembrane cell, the mirtazapine transdermal permeability from each of the transdermal patches of Examples 7 to 15 obtained according to the above-mentioned formulation and production method was investigated. A skin sample taken from the back of a hairless mouse (n = 3 or 4, Hr-/Kud, Kyudo) was fixed to the transmembrane cell, then the patch of each Example was stuck to the side of the horny layer of the skin, and thereafter this was set in an in-vitro membrane permeation tester. An aqueous 40% polyethylene glycol 400 solution was used as the receptor liquid, and the amount of mirtazapine having transferred into the receptor liquid was measured. HPLC was used for the quantitation of mirtazapine. From the inclination of the regression expression obtained from the accumulated mirtazapine permeation data derived from the test results and the sampling time through computation according to the method of least squares, the transdermal permeation rate was determined. The results are shown in Table 4.

**[Table 4]**

| | Transdermal Permeation Rate in Steady State (µg/cm²/h) |
|---|---|
| Example 7 | 16.4 |
| Example 8 | 21.8 |
| Example 9 | 18.2 |
| Example 10 | 19.5 |
| Example 11 | 13.0 |
| Example 12 | 21.2 |
| Example 13 | 25.1 |
| Example 14 | 20.4 |
| Example 15 | 22.8 |

As obvious from Table 4, the transdermal patches of the invention all exhibited mirtazapine releasability; and the transdermal permeation rate in steady state of the patches of Examples 7 to 15 was from 13.0 to 25.1 µg/cm²/hr. Further, the patches of Example 8, Example 12, Example 13, Example 14 and Example 15 were recognized to have increased transdermal permeability owing to the transdermal absorption promoter therein. Of those, the cases where any of myristate, sebacate, menthol, polyoxyethylene oleyl ether and Polysorbate 80™ was used as the absorption promoter suggested the improvement of the transdermal permeability even though the amount of the absorption promoter was from 1 to 10% by mass and was relatively small.

### Test Example 2

### Measurement of Brain Monoamine Level, and Measurement of Plasma Mirtazapine Level:

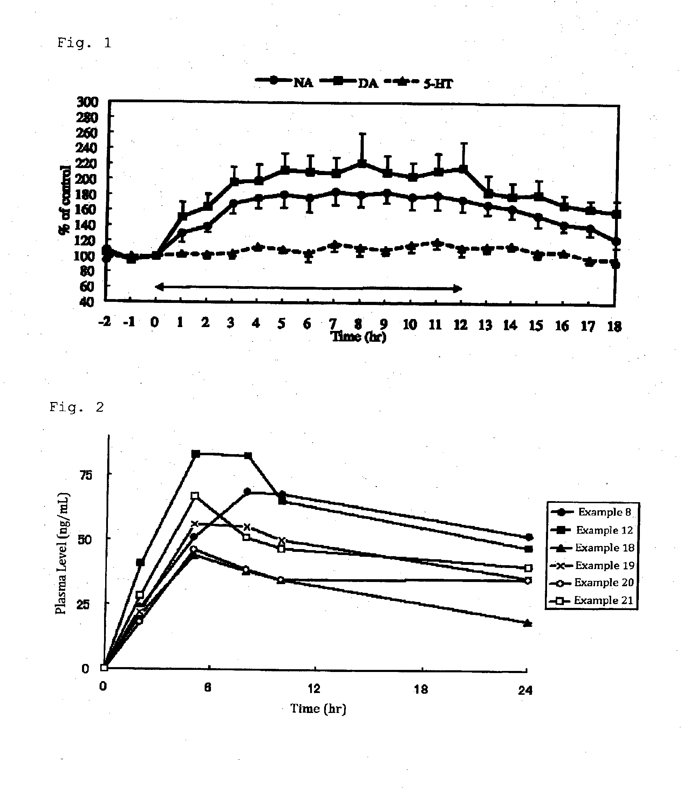

Rats (male, body weight: 200 to 300 g, n = 5) were anesthetized by inhalation of isoflurane, and then a cannula for long-term continuous administration was buried under the skin of each rat and a guide (microdialysis) was in the prefrontal region thereof. In 5 to 7 days after the guide insertion, a probe for measurement of monoamine in the brain was inserted into the prefrontal region, and via the microsyringe pump connected to the probe, a perfusate was perfused at a flow rate of 0.5 µL/min. For mirtazapine administration, a mirtazapine 3 mg/mL/kg solution was administered in a predetermined dose via the microsyringe pump, taking 12 hours. A Ringer's solution was used as the perfusate, which was collected at intervals of 1 hour using a fraction collector. The results are shown in Fig. 1.

Next, at the same dose mode and through the same administration route as above, mirtazapine (3 mg/mL/kg) was administered in a predetermined dose, taking 12 hours, and in 6 hours after the start, the blood was collected. The time at which the administration was started was referred to as the test start time. Heparin was added to the collected blood and then centrifuged, and the thus-separated plasma was the sample.

Monoamine was measured from the perfusate collected by the fraction collector, and mirtazapine was measured from the plasma sample through HPLC.

As shown in Fig. 1, increase in NA and DA was admitted in 1 hour after the start of mirtazapine administration in the rat prefrontal region; NA increased up to 184.7 ± 17.3% (mean ± SEM) in 7 hours after the start of administration; DA increased up to 222.1 ± 38.5% in 8 hours after the start of administration; and the data confirm the NA and DA promotion effect attained by mirtazapine administration. Regarding the increase in NA and DA, NA decreased down to 124.2 ± 10.7 and DA to 159.2 ± 15.6% in 18 hours after the start of administration. However, no influence on 5-HT was admitted. The mean concentration of mirtazapine in the rat plasma in 6 hours after the start of administration was 27.5 ± 3.5 ng/mL (mean ± SEM).

### Test Example 3

### Kinetic Study in Rat Blood:

10 cm² of the transdermal patches of Example 8 and Example 12, 20 cm² of the transdermal patches of Example 18 and Example 19, and 5 cm² of the transdermal patches of Example 20 and Example 21 were applied to the hair-shaved flank of rats (male, body weight: 200 to 230 g, n = 3 or 4). The time at which the test substance was applied was referred to as the test start time, and in 24 hours after the test start time, the transdermal patches were removed. In 2, 5, 8, 10 and 24 hours after the test start time, blood was collected. The collected blood was centrifuged, and the separated plasma was taken as a sample. The mirtazapine concentration in the sample blood was measured through HPLC. The results are shown in Fig. 2.

As obvious from Fig. 2, the plasma level, Cmax in Example 8, Example 12, Example 18, Example 19, Example 20 and Example 21 was from 40 to 85 ng/mL, respectively. The results in the previous Test Example 2 confirmed monoamine increase of at least 27.5 ng/mL as the plasma level. The present results suggest that the mirtazapine content of from 2 to 30% by mass relative to the drug-containing layer of the transdermal patch of the invention secures increase in NA and DA through transdermal mirtazapine administration, or that is, the transdermal patch of the invention enables transdermal absorption of a therapeutically-effective amount of the active ingredient, and that the invention provides a mirtazapine-containing transdermal patch capable of stably sustaining an effective blood level of the active ingredient.

### Test Example 4

According to the measurement method mentioned below, the patches obtained in Examples 1 to 6, Example 9, Example 10, Example 12 and Comparative Example 1 were analyzed with time for the amount of mirtazapine, related substance 1 and related substance 2. The related substance 1 and the related substance 2 are detected in a retention time of about 7 minutes and a retention time of about 11 minutes, respectively, in analysis under the condition mentioned below.

The test sample cut in a size of 5 cm² was individually wrapped with an aluminium wrapping material, stored at 60°C and analyzed with time for the amount of mirtazapine, related substance 1 and related substance 2 therein.

The release sheet of each test sample was peeled off, the test sample was taken in a 50-mL centrifugal tube, 10 mL of tetrahydrofuran was added thereto and shaken for 20 minutes to completely dissolve the patch material. 10 mL of water/methanol (2/1, v/v) was added to the resulting liquid, shaken for 20 minutes to thereby completely aggregate and precipitate the adhesive base ingredient. The supernatant was quantified through high-performance liquid chromatography.

The operation of high-performance liquid chromatography was as follows: As the column, used was a stainless tube having an inner diameter of 3.0 mm and a length of 7.5 cm, which was filled with octadecylsilylated silica gel (3 µm) for liquid chromatography. The detection wavelength was 251 nm. As the mobile phase, used were water/trifluoroacetic acid (2000/1, v/v) (mobile phase A) and acetonitrile/trifluoroacetic acid (2000/1, v/v) for liquid chromatography (mobile phase B). For mobile phase supply, the mobile phase A and the mobile phase B were mixed under a desired condition to provide a controlled concentration gradient. The results are shown in Table 5.

**[Table 5]**

| Sample | Detected Substance | Amount of Related Substances (%)*¹ | | |
|---|---|---|---|---|
| | | Day 0 | Day 7 | Day 14 |
| Example 1 | Mirtazapine | 99.86 | 99.67 | 99.62 |
| | Related substance 1 | 0.14 | 0.24 | 0.26 |
| | Related substance 2 | 0.00 | 0.09 | 0.12 |
| Example 2 | Mirtazapine | 99.92 | 99.76 | 99.73 |
| | Related substance 1 | 0.08 | 0.18 | 0.20 |
| | Related substance 2 | 0.00 | 0.06 | 0.07 |
| Example 3 | Mirtazapine | 100.00 | 99.77 | 99.65 |
| | Related substance 1 | 0.00 | 0.15 | 0.23 |
| | Related substance 2 | 0.00 | 0.08 | 0.12 |
| Example 4 | Mirtazapine | 100.00 | 99.85 | 99.79 |
| | Related substance 1 | 0.00 | 0.15 | 0.19 |
| | Related substance 2 | 0.00 | 0.00 | 0.02 |
| Example 5 | Mirtazapine | 99.81 | 99.71 | 99.68 |
| | Related substance 1 | 0.19 | 0.19 | 0.17 |
| | Related substance 2 | 0.00 | 0.10 | 0.15 |
| Example 6 | Mirtazapine | 99.82 | 99.50 | 99.31 |
| | Related substance 1 | 0.18 | 0.35 | 0.48 |
| | Related substance 2 | 0.00 | 0.15 | 0.21 |
| Example 9 | Mirtazapine | 100.00 | 99.83 | 99.80 |
| | Related substance 1 | 0.00 | 0.13 | 0.13 |
| | Related substance 2 | 0.00 | 0.04 | 0.07 |
| Example 10 | Mirtazapine | 100.00 | 99.83 | 99.81 |
| | Related substance 1 | 0.00 | 0.12 | 0.14 |
| | Related substance 2 | 0.00 | 0.05 | 0.05 |
| Example 12 | Mirtazapine | 100.00 | 99.85 | 99.81 |
| | Related substance 1 | 0.00 | 0.13 | 0.13 |
| | Related substance 2 | 0.00 | 0.02 | 0.06 |
| Comparative Example 1 | Mirtazapine | 99.83 | 99.60 | 99.46 |
| | Related substance 1 | 0.17 | 0.30 | 0.37 |
| | Related substance 2 | 0.00 | 0.10 | 0.17 |

| | | | | |
|---|---|---|---|---|
| *1: The peak areas of mirtazapine, the related substance 1 and the related substance 2 were summed up to be 100%, and the data (n = 3) were averaged to be the mean value shown in the Table. | | | | |

As obvious from Table 5, the related substance 1 and the related substance 2 were formed in Comparative Example 1 not containing an antioxidant, and in Examples 1 to 5, Example 9, Example 10 and Example 12, in which any one or two of ascorbyl palmitate, dibutylhydroxytoluene and isoascorbic acid were incorporated in the transdermal patch containing mirtazapine or its salt, the formations of the related substance 1 and the related substance 2 were significantly retarded as compared with that in Comparative Example 1. Above all, in Example 4 containing both L-ascorbyl palmitate and dibutylhydroxytoluene, the amount of the related substance 1 formed was about a half in Comparative Example 1 and the amount of the related substance 2 formed was about 1/10 in Comparative Example 1; and the data suggest that these antioxidants are effective for preventing the formation of related substances in the mirtazapine-containing transdermal patch.

### Industrial Applicability

As described above, the transdermal patch of the invention is a novel patch that contains the NaSSA mirtazapine as the active ingredient therein and enables quantitative transdermal drug administration. The transdermal patch of the invention is free from any rapid blood level increase that is recognized with oral preparations, while exhibiting a sufficient therapeutical effect, and is therefore expected to prevent side effects. Further, the transdermal patch of the invention can be stored for a long period of time, not undergoing decomposition of the effective ingredient NaSSA (mirtazapine), and therefore enables effective and sustainable utilization of the pharmacological effect of the active ingredient therein. Consequently, the transdermal patch of the invention is a preparation effective for treatment for depression. Further, in use thereof, the transdermal patch of the invention can be differentiated from any other transdermal patches containing an antidepressant that has a different functional mechanism such as SSRI or the like, and therefore broadens the patients' latitude in selecting desired pharmaceutical preparations in treatment for depression, and is therapeutically useful.

## Claims

1. A transdermal patch comprising a support, a drug-containing layer and a release liner, wherein the drug-containing layer is a matrix-type adhesive layer that contains one or more adhesive base materials, and wherein the drug-containing layer contains a noradrenergic and specific serotonergic antidepressant as the active ingredient and an antioxidant therein,
wherein the noradrenergic and specific serotonergic antidepressant is mirtazapine or a pharmaceutically-acceptable salt thereof.

2. The transdermal patch as claimed in claim 1, wherein the content of the noradrenergic and specific serotonergic antidepressant is from 2 to 30% by mass relative to the drug-containing layer.

3. The transdermal patch as claimed in claim 1, wherein the content of the noradrenergic and specific serotonergic antidepressant is from 3 to 25% by mass relative to the drug-containing layer.

4. The transdermal patch as claimed in any one of claims 1 to 3, which contains one or more adhesive base materials selected from a rubber-type adhesive base, an acrylic polymer and a silicone polymer, as the base ingredient in the matrix-type adhesive layer.

5. The transdermal patch as claimed in any of claims 1 to 4, wherein the drug-containing layer further contains a transdermal absorption promoter.

6. The transdermal patch as claimed in claim 5, wherein the content of the transdermal absorption promoter is from 1 to 10% by mass relative to the drug-containing layer.

7. The transdermal patch as claimed in claim 5 or 6, wherein the transdermal absorption promoter is at least one selected from myristates, sebacates, menthol, polyoxyethylene oleyl ether and Polysorbate 80™.

8. The transdermal patch as claimed in claim 1, which contains, as the antioxidant therein, one or more antioxidants selected from ascorbic acid and its ester, phenolic antioxidants and 2-mercaptobenzimidazole.

9. The transdermal patch as claimed in claim 8, wherein the phenolic antioxidant is dibutylhydroxytoluene.

10. The transdermal patch as claimed in claim 8 or 9, wherein ascorbic acid or its ester is L-ascorbyl palmitate and/or isoascorbic acid.

11. The transdermal patch as claimed in any of claims 8 to 10, which contains, as the antioxidant therein, L-ascorbyl palmitate in an amount of from 0.02 to 0.2% by mass relative to the drug-containing layer and dibutylhydroxytoluene in an amount of from 0.1 to 1% by mass relative to the drug-containing layer.

12. The transdermal patch as claimed in any of claims 1 to 11, wherein the drug-containing layer is a matrix-type adhesive layer that contains the base ingredient, the noradrenergic and specific serotonergic antidepressant and the antioxidant in a dissolved state.

13. The transdermal patch as claimed in claim 12, which contains one or more adhesive base materials selected from a rubber-type adhesive base, an acrylic polymer and a silicone polymer, as the base ingredient in the matrix-type adhesive layer.

14. The transdermal patch as claimed in any of claims 1 to 13, wherein a rubber-type adhesive base material is incorporated as the base ingredient in the matrix-type adhesive layer.

## Patentansprüche

1. Transdermales Pflaster umfassend eine Auflage, eine arzneimittelhaltige Schicht und eine Abziehschicht, worin die arzneimittelhaltige Schicht eine Adhäsionsschicht vom Matrix-Typ ist, die einen oder mehrere adhäsive Grundmaterialien enthält, und worin die arzneimittelhaltige Schicht ein noradrenerges und spezifisch serotonerges Antidepressivum als aktiven Inhaltsstoff und ein Antioxidans enthält,
worin das noradrenerge und spezifisch serotonerge Antidepressivum Mirtazapin oder ein pharmazeutisch verträgliches Salz davon ist.

2. Das transdermale Pflaster wie in Anspruch 1 beansprucht, worin der Gehalt des noradrenergen und spezifisch serotonergen Antidepressivums 2 bis 30 Gewichts% bezogen auf die arzneimittelhaltige Schicht beträgt.

3. Das transdermale Pflaster wie in Anspruch 1 beansprucht, worin der Gehalt des noradrenergen und spezifisch serotonergen Antidepressivums 3 bis 25 Gewichts% bezogen auf die arzneimittelhaltige Schicht beträgt.

4. Das transdermale Pflaster wie in einem der Ansprüche 1 bis 3 beansprucht, das ein oder mehrere adhäsive Grundmaterialien ausgewählt aus einem Adhäsivgrundstoff vom Gummi-Typ, einem Acrylpolymer und einem Silikonpolymer als Grundstoff in der Adhäsionsschicht vom Matrix-Typ enthält.

5. Das transdermale Pflaster wie in einem der Ansprüche 1 bis 4 beansprucht, worin die arzneimittelhaltige Schicht ferner einen transdermalen Absorptionspromotor enthält.

6. Das transdermale Pflaster wie in Anspruch 5 beansprucht, worin der Gehalt am transdermalen Absorptionspromotor von 1 bis 10 Gewichts-% bezogen auf die arzneimittelhaltige Schicht enthält.

7. Das transdermale Pflaster wie in Anspruch 5 oder 6 beansprucht, worin der transdermale Absorptionspromotor mindestens einer ist ausgewählt aus Myristaten, Sebacaten, Menthol, Polyoxyethylen-Oleyl-Ether und Polysorbat 80™.

8. Das transdermale Pflaster wie in Anspruch 1 beansprucht, das als das darin enthaltene Antioxidans ein oder mehr Antioxidantien ausgewählt aus Ascorbinsäure und deren Ester, phenolischen Antioxidantien und 2-Mercaptobenzimidazol enthält.

9. Das transdermale Pflaster gemäß Anspruch 8, worin das phenolische Antioxidans Dibutylhydroxytoluol ist.

10. Das transdermale Pflaster wie in Anspruch 8 oder 9 beansprucht, worin Ascorbinsäure oder deren Ester L-Ascorbylpalmitat und/oder Isoascorbinsäure ist.

11. Das transdermale Pflaster wie in einem der Ansprüche 8 bis 10 beansprucht, das als das darin enthaltene Antioxidans L-Ascorbylpalmitat in einer Menge von 0,02 bis 0,2 Gewichts-% bezogen auf die arzneimittelhaltige Schicht und Dibutylhydroxytoluol in einer Menge von 0,1 bis 1 Gewichts-% bezogen auf die arzneimittelhaltige Schicht enthält.

12. Das transdermale Pflaster wie in einem der Ansprüche 1 bis 11 beansprucht, worin die arzneimittelhaltige Schicht eine Adhäsionsschicht vom Matrix-Typ ist, die das Grundmaterial, das noradrenerge und spezifisch serotonerge Antidepressivum und das Antioxidans in einem gelösten Zustand enthält.

13. Das transdermale Pflaster gemäß Anspruch 12, das ein oder mehrere adhäsive Grundmaterialien ausgewählt aus einem Adhäsivgrundstoff vom Gummi-Typ, einem Acrylpolymer und einem Silikonpolymer als Grundstoff in der Adhäsionsschicht vom Matrix-Typ enthält.

14. Das transdermale Pflaster wie in einem der Ansprüche 1 bis 13 beansprucht, worin ein Adhäsivgrundstoff vom Gummi-Typ als Grundstoff in die Adhäsionsschicht vom Matrix-Typ inkorporiert wird.

## Revendications

1. Timbre transdermique comprenant un support, une couche contenant un médicament et une doublure antiadhésive, dans lequel la couche contenant un médicament est une couche adhésive de type matrice qui contient un ou plusieurs matériau(x) de base adhésif(s), et dans lequel la couche contenant un médicament contient dans celle-ci un antidépresseur noradrénergique et sérotoninergique spécifique à titre d'agent actif et un antioxydant,
dans lequel l'antidépresseur noradrénergique et sérotoninergique spécifique est la mirtazapine ou un sel pharmaceutiquement acceptable de celle-ci.

2. Timbre transdermique selon la revendication 1, dans lequel la teneur en l'antidépresseur noradrénergique et sérotoninergique spécifique est de 2 à 30 % en masse par rapport à la couche contenant un médicament.

3. Timbre transdermique selon la revendication 1, dans lequel la teneur en l'antidépresseur noradrénergique et sérotoninergique spécifique est de 3 à 25 % en masse par rapport à la couche contenant un médicament.

4. Timbre transdermique selon l'une quelconque des revendications 1 à 3, qui contient un ou plusieurs matériau(x) de base adhésif(s) choisi(s) parmi une base adhésive de type caoutchouc, un polymère acrylique et un polymère siliconé, en tant qu'ingrédient de base dans la couche adhésive de type matrice.

5. Timbre transdermique selon l'une quelconque des revendications 1 à 4, dans lequel la couche contenant un médicament contient en outre un promoteur d'absorption transdermique.

6. Timbre transdermique selon la revendication 5, dans lequel la teneur en le promoteur d'absorption transdermique est de 1 à 10 % en masse par rapport à la couche contenant un médicament.

7. Timbre transdermique selon la revendication 5 ou 6, dans lequel le promoteur d'absorption transdermique est au moins l'un parmi les myristates, les sébaçates, le menthol, l'oléyléther polyoxyéthyléné et le Polysorbate 80™.

8. Timbre transdermique selon la revendication 1, qui contient, à titre d'antioxydant, un ou plusieurs antioxydant(s) choisi(s) parmi l'acide ascorbique et ses esters, les antioxydants phénoliques et le 2-mercaptobenzimidazole.

9. Timbre transdermique selon la revendication 8, dans lequel l'antioxydant phénolique est le dibutylhydroxytoluène.

10. Timbre transdermique selon la revendication 8 ou 9, dans lequel l'acide ascorbique ou son ester est le palmitate de L-ascorbyle et/ou l'acide isoascorbique.

11. Timbre transdermique selon l'une quelconque des revendications 8 à 10, qui contient, à titre d'antioxydant, du palmitate de L-ascorbyle en une quantité de 0,02 à 0,2 % en masse par rapport à la couche contenant un médicament, et du dibutylhydroxytoluène en une quantité de 0,1 à 1 % en masse par rapport à la couche contenant un médicament.

12. Timbre transdermique selon l'une quelconque des revendications 1 à 11, dans lequel la couche contenant un médicament est une couche adhésive de type matrice qui contient l'ingrédient de base, l'antidépresseur noradrénergique et sérotoninergique spécifique et l'antioxydant à l'état dissous.

13. Timbre transdermique selon la revendication 12, qui contient un ou plusieurs matériau(x) de base adhésif(s) choisi(s) parmi une base adhésive de type caoutchouc, un polymère acrylique et un polymère siliconé, en tant qu'ingrédient de base dans la couche adhésive de type matrice.

14. Timbre transdermique selon l'une quelconque des revendications 1 à 13, dans lequel un matériau de base adhésif de type caoutchouc est incorporé en tant qu'ingrédient de base dans la couche adhésive de type matrice.
